Europäisches Patentamt

(19) **European Patent Office**

**Office européen des brevets**

(11) Publication number: **0 013 826**
**A1**

## (12) EUROPEAN PATENT APPLICATION

(21) Application number: **79303005.7**

(22) Date of filing: **21.12.79**

(51) Int. Cl.³: **C 07 C 103/52, A 61 K 37/26 // B01D15/04, B01J39/22**

(30) Priority: **26.12.78 US 972744**

(43) Date of publication of application: **06.08.80 Bulletin 80/16**

(84) Designated Contracting States: **DE GB NL SE**

(71) Applicant: **ELI LILLY AND COMPANY, 307, East McCarty Street, Indianapolis Indiana (US)**

(72) Inventor: **Jackson, Richard Lee, 951 N. Arlington Avenue, Indianapolis Indiana 46219 (US)**

(74) Representative: **McVey, Kenneth William Henry et al, Erl Wood Manor, Windlesham Surrey, GU20 6PH (GB)**

(54) **Process for purifying insulin and insulin so prepared.**

(57)   This invention relates to a process for purifying insulin using a cross-linked dextran gel cation exchanger containing sulfoalkyl or carboxymethyl groups. The process comprises:

a) swelling and equilibrating a cross-linked dextran gel cation exchanger containing sulfoalkyl or carboxymethyl groups;

b) packing a column with the swollen gel;

c) dissolving a partially purified insulin at a concentration in the range of from about 0.5% to about 12%, weight per volume, in an acidic aqueous medium having a pH of from about 2.5 to about 4.5 and containing urea in a concentration of from about 4 to about 8 moles per liter of medium;

d) bringing the insulin solution into contact with the column-packed cation exchanger gel; and

e) eluting insulin from the cation exchanger gel at a temperature of from about 5 °C. to about 25 °C. by passing an eluant over the insulin-containing cation exchanger gel, said eluant comprising acidic aqueous medium containing inorganic salt, and said inorganic salt being present in said acidic aqueous medium in a progressively increasing concentration commencing at or below about 0.2 moles per liter and extending to as high as about 0.4 moles per liter.

## Process for Purifying Insulin

This invention relates to the production of insulin. More particularly, this invention relates to the production of high-purity insulin by the use of a cation-exchange gel.

Since its discovery in 1921 as a component of the pancreas, insulin has assumed world-wide importance in the treatment of diabetes mellitus. Because the extraction procedures employed to remove insulin from pancreas tissue also remove substantial amounts of noninsulin protein, considerable effort has been expended to discover and develop methods for purifying insulin, i.e., methods for separating insulin from noninsulin protein.

One of the earliest methods exploited the phenomenon that a protein has minimum solubility at its isoelectric point. Banting et al. disclosed in U.S. Patent No. 1,469,994 a purification process which comprises precipitating at their isoelectric point noninsulin proteins present in an aqueous extract from the pancreas.

Walden, in U.S. Patent No. 1,520,673, disclosed the isoelectric precipitation of insulin from an aqueous pancreatic extract at about pH 4 to about pH 7, preferably between pH 4.5 and pH 5.5.

In another early method, precipitation of insulin was effected by the addition of a sufficient amount of an inorganic salt. Muslin, in U.S. Patent No. 1,547,515, disclosed the first process for salting out insulin from an extraction solvent by the addition of sodium chloride.

Lautenschläger and Lindner, in U.S. Patent No. 2,449,076, described a differential salting out process for insulin purification. The method comprises salting out insulin from a neutralized extract by the addition of sodium chloride, filtering, redissolving the residue, and again salting out the insulin using a lower sodium chloride concentration.

Another method for purifying insulin involves the precipitation or crystallization of insulin from solution as a zinc insulin complex. In general, zinc insulin is precipitated from a buffered, aqueous solution containing zinc ions. Various buffers have been employed. Peterson, for example, in U.S. Patent No. 2,626,228, used a citric acid-citrate buffer.

Homan, in U.S. Patent No. 2,663,666, disclosed a method of insulin purification comprising sequentially adjusting the pH of an acidic aqueous pancreatic extract to pH 4.0-5.5, pH 7.8-8.5, and pH 3.0-3.5; filtering the resulting mixture after each adjustment; and discarding the residue consisting of undesired acidic and basic components of the extract, from each filtration. Insulin then is recovered from the final filtrate.

Present commercial processes for the purification of insulin can comprise at least three of the above procedures, including, typically, one or more salt precipitations at differing salt concentration, one or more isoelectric precipitations, and at least one zinc crystallization.

Jorpes et al., in U.S. Patent No. 2,878,159, disclosed a method for the purification of insulin by passing an aqueous pancreatic extract over a carboxylic acid type cation-exchange resin. Bolini et al., in U.S. Patent No. 3,069,323, disclosed the use of an aminocellulose anion-exchange resin in a similar process.

British Patent No. 1,054,523 describes a method for preparing crystalline insulin from aqueous alcoholic pancreatic extracts or aqueous solutions of insulin by contacting them with a sulfonated cation-exchange resin at a pH greater than 7.4 or lower than 3.5. In addition, from U.S. Patent No. 3,221,008, zinc insulin is subjected to column chromatography on the cation exchanger "Dowex 50/X1" which is a sulfonated styrene-divinylbenzene copolymer in bead form.

Japanese Patent No. 6492/63 and Belgian Patent No. 676,879 both disclose the use of a carboxymethylcellulose in the purification of insulin.

In addition to the aforementioned uses of both cation and anion exchange resins and carboxymethylcellulose substrates in the treatment of insulin, dextran gels have also been employed. Thus, German Patent No. 2,212,695 employs what is described as an epichlorohydrin cross-linked dextran gel, the hydroxyl groups of which are for the most part alkylated. Furthermore, as described in United States Patent Nos. 3,876,623 and 3,878,186, gel filtration has been used in the purification of alkali metal or ammonium insulins.

U.S. Patent No. 3,097,676 discloses treating insulin on an anion-exchanger chromatographic column and eluting it therefrom with an eluant containing water together with a water-miscible monohydric aliphatic alcohol.

Although, as indicated above, the literature references to the chromatographic purification of insulin have been rather plentiful, the need remains for a process which will permit production of high purity insulin by removal of non-insulin proteins such as proinsulin, proinsulin-like proteins, somatostatin, glucagon, and the like, while at the same time minimizing insulin loss. Proinsulin and proinsulin-like proteins are the most prevalent noninsulin components present in commercial insulins. Although these components generally constitute less than about 8 weight percent of commercial zinc insulins, some of these components are believed to be antigenic or immunogenic in nature. See, for example, Chance et al., Science, 161, 165 (1968); Steiner et al., Diabetes, 18, 725 (1968); Bromer, BioScience, 20, 702 (1970); and Rubenstein et al., Ann. Rev. Med., 22, 1 (1971). Therefore, a continuing need is the discovery of a facile process for the removal on a commercial scale of non-insulin proteins from insulin with minimal insulin loss.

The term "insulin" as used herein includes not only insulin per se, but all insulin-like proteins, such as desamido insulin. Because insulin and insulin-like proteins have similar hypoglycemic activities, it usually is not required to separate insulin from all other pancreatic proteins. In other words, a homogeneous insulin normally is not required.  Nevertheless, as indicated above, it is highly desirable to separate on a large scale non-insulin proteins, such as proinsulin, proinsulin-like proteins, somatostatin, glucagon, and the like, from insulin to obtain a commercial product having minimal potential antigenic or immunogenic properties.

It is to such an end that the process of this invention is directed.  Although certain prior art processes do permit a measure of separation of non-insulin proteins from insulin, such generally is accomplished only with loss of substantial quantities of insulin product.  The process of this invention permits the production of highly purified insulin on a large scale and with minimal insulin loss.

It therefore is an object of this invention to provide a process for the purification of insulin using a cation exchange gel under conditions which provide both high insulin recovery and substantially reduced amounts of proinsulin and other contaminating proteins.

Thus, this invention is directed to a process for increasing the purity of insulin, which comprises

a)   swelling and equilibrating a cross-linked dextran gel cation exchanger containing sulfoalkyl or carboxymethyl groups;

b)   packing a column with the swollen gel;

c)   dissolving a partially purified insulin at a concentration in the range of from about 0.5% to about 12%, weight per volume, in an acidic aqueous medium having a pH of from about 2.5

X-5317A -6-

0013826

to about 4.5 and containing urea in a concentration of from about 4 to about 8 moles per liter of medium;

d) bringing the insulin solution into contact with the column-packed cation exchanger gel; and

e) eluting insulin from the cation exchanger gel at a temperature of from about 5°C. to about 25°C. by passing an eluant over the insulin-containing cation exchanger gel, said eluant comprising acidic aqueous medium containing inorganic salt, and said inorganic salt being present in said acidic aqueous medium in a progressively increasing concentration commencing at or below about 0.2 moles per liter and extending to as high as about 0.4 moles per liter.

In addition, in order to achieve an even greater degree of insulin purity, the insulin subjected to purification in accordance with the foregoing can be that obtained from treatment under defined conditions using an anion exchange gel.

Thus, a further aspect of this invention is a process for increasing the purity of insulin, which comprises

a) swelling and equilibrating a cross-linked dextran gel anion exchanger;

b) packing a column with the swollen anion exchanger gel;

c)  dissolving a partially purified insulin at a concentration in the range of from about 0.5% to about 12%, weight per volume, in a neutral or moderately alkaline aqueous medium having a pH of from about 7 to about 9 and containing urea in a concentration of from about 4 to about 8 moles per liter;

d)  bringing the insulin solution into contact with the anion exchanger gel;

e)  eluting insulin from the anion exchanger gel at a temperature in the range of from about 5°C. to about 10°C. by passing an eluant over the insulin-containing anion exchanger gel, said eluant comprising neutral or moderately alkaline aqueous medium containing inorganic salt, and said inorganic salt being present in said neutral or moderately alkaline aqueous medium in a progressively increasing concentration extending to as high as about 0.2 moles per liter;

f)  swelling and equilibrating a cross-linked dextran gel cation exchanger containing sulfoalkyl or carboxymethyl groups;

g)  packing a column with the swollen cation exchange gel;

h)  bringing insulin, obtained from step (e) and dissolved at a concentration in the range of from about 0.5% to about 12%, weight per volume, in an acidic aqueous medium having a pH of from about 2.5 to about 4.5 and containing urea in a concentration of from about 4 to about 8 moles per liter of medium, into contact with the cation exchanger gel; and

i) eluting insulin from the cation exchanger gel at a temperature of from about 5°C. to about 25°C. by passing an eluant over the insulin-containing cation exchanger gel, said eluant comprising acidic aqueous medium containing inorganic salt, and said inorganic salt being present in said acidic aqueous medium in a progressively increasing concentration commencing at or below about 0.2 moles per liter and extending to as high as about 0.4 moles per liter.

The insulin that is subjected to purification by the process of this invention is one which has been partially purified, for example, by salt precipitation, isoelectric precipitation, and/or zinc or alkali metal crystallization. Generally, the insulin which is treated by the process of this invention will have a purity of at least about 80%, and, more likely, it will have an even greater level of purity, i.e., a purity of about 90% or even greater.

The process of this invention is directed to a cation exchange treatment or, in tandem, an anion exchange treatment followed by a cation exchange treatment. The cation exchanger is a cross-linked dextran gel which carries strongly acidic functional groups. Such groups, in particular, include sulfoalkyl or carboxymethyl groups, and, of the sulfoalkyl groups, more particularly, those having from 1 to 3 carbon atoms. The preferred cross-linked dextran gels include the Sephadex series (from Pharmacia Fine Chemicals, Inc., Piscataway, New Jersey). This series is specifically designated CM-Sephadex, which carries carboxymethyl groups as the functional groups, or SP-Sephadex, which carries sulfopropyl groups as the functional groups.

The process of this invention is carried out using a chromatographic column. The type of column which is employed is not critical. The choice of column height, diameter, or configuration will depend upon the particular operating parameters which are desired.

Insulin purification generally is carried out at a loading ranging from about 1 to about 120 gms. per liter of swollen and equilibrated cation exchanger and, preferably, ranging from about 50 to about 100 gms. per liter. Of course, optimal conditions can be readily determined for any particular system.

In carrying out the process of this invention, the insulin to be treated generally is dissolved in an acidic aqueous medium of defined composition and characteristics. The acidic aqueous medium contains an acid in a quantity affording a final pH within the range of from about 2.5 to about 4.5. Preferably, the final pH is from about 3.2 to about 4.2, and, most preferably, is from about 3.6 to about 4.2. Specific preferred final pH's are 3.6 and 4.2. Suitable acids include, among others, hydrochloric acid, phosphoric acid, formic acid, acetic acid, propionic acid, n-butyric acid, isobutyric acid, pentanoic acid, and the like. Preferably, the acid is selected from acetic acid, hydrochloric acid, and phosphoric acid, and, of the three, acetic acid is most preferred.

In addition to the acid, the acidic aqueous medium contains urea. The urea is present in an amount such that the final concentration of urea is from about 4 to about 8 moles per liter. Preferably, the concentration of urea ranges from about 7 to about 8 moles per liter, and, most preferably, is about 7 moles per liter.

The insulin, dissolved in an acidic aqueous medium at a concentration in the range of from about 0.5% to about 12%, weight per volume, is brought into contact with the cation exchange gel which has previously been swollen and equilibrated, preferably by contact with a quantity of acidic aqueous medium which may contain a small amount, up to about 0.15 moles per liter, of inorganic salt.

The insulin then is eluted from the cation exchange gel. The elution is carried out at a temperature in the range of from about 5°C. to about 25°C., and, preferably, at about 5°C. The eluting medium (eluant) comprises a mixture of acidic aqueous medium and an inorganic salt. Although it is not essential, the acidic aqueous medium which makes up part of the eluant may be the same as the acidic aqueous medium employed in applying the insulin to the cation exchange gel. The inorganic salt, which represents the other component of the eluant, can be any of a wide variety of salts. Typical inorganic salts are sodium chloride, potassium chloride, ammonium chloride, and the like. However, the preferred salt is sodium chloride.

The eluting medium, comprising a mixture of acidic aqueous medium and inorganic salt, is brought into contact with the cation exchange gel, and the insulin is eluted therefrom. The inorganic salt is provided as a salt gradient such that the concentration of salt in the acidic aqueous medium delivered to the cation exchange gel increases on a step-wise or a continuous basis. The salt concentration generally is increased progressively over a range which is within the range of from at or below about 0.2 mole per liter to about 0.4 mole per liter, or, preferably, to about 0.36 mole per liter, or, most preferably, until elution of the insulin has been achieved. The latter generally will occur before either of the aforementioned upper limits of salt concentration has been reached.

A particularly preferred embodiment of the insulin purification process of this invention involves subjecting the insulin to purification by an anion exchanger gel process prior to its being treated in accordance with the cation exchanger gel process of this invention. This sequence itself represents another facet of this invention.

In accordance with this sequence, the insulin, having a purity as aforedescribed for the cation exchanger treatment, is subjected to purification using an anion exchange gel.

The anion exchanger is a cross-linked dextran gel which carries either weakly basic or strongly basic groups. (Examples of suitable anion exchanger gels are those of the Sephadex series available from Pharmacia Fine Chemicals, Inc., Piscataway, New Jersey). In particular, DEAE-Sephadex, which is a cross-linked dextran gel containing diethylaminoethyl groups, is a weakly basic anion exchanger gel, and QAE-Sephadex, which is a cross-linked dextran gel containing diethyl-(2-hydroxypropyl)aminoethyl groups, is a strongly basic anion exchanger gel. Of the two, DEAE-Sephadex is preferred.

The anion exchanger gel treatment also is carried out using a chromatographic column, generally under loading conditions such as those described hereinbefore for the cation exchange treatment.

In carrying out the anion exchange step of the two stage insulin purification process of this invention, the insulin to be purified is dissolved in a buffered neutral or alkaline aqueous medium containing from about 4 to about 8 moles of urea per liter and carrying a final pH of from about 7 to about 9.

The buffered medium can be prepared using any of a wide variety of available buffers. Examples of suitable buffers include glycine-sodium hydroxide, tris(hydroxymethyl)aminomethane, and the like. Normally, the buffered medium will be prepared by dissolving the selected buffer in water, adding urea to afford the desired concentration, and, if necessary, adjusting the solution to the desired pH by addition, for example, of a sufficient quantity of an acid such as hydrochloric acid.

As noted, the buffer medium will contain urea in an amount of from about 4 to about 8 moles per liter. Preferably, the concentration of urea ranges from about 7 to about 8 moles per liter, and, most preferably, is present at a concentration of about 7 moles per liter.

Moreover, the final pH of the buffer medium is from about 7 to about 9. Preferably, the final pH is from about 8.0 to about 8.5, and, most preferably, is about 8.1.

The insulin, dissolved in the buffered neutral or alkaline aqueous medium at a concentration in the range of from about 0.5% to about 12%, weight per volume, is brought into contact with the anion exchange gel which has previously been swollen and equilibrated, preferably by contact with a quantity of the selected buffer medium.

The insulin then is eluted from the anion exchanger gel. The elution is carried out at a temperature in the range of from about 5°C. to about 10°C., and, preferably, at about 5°C. The eluting medium (eluant) comprises a mixture of buffered neutral or alkaline aqueous medium and an inorganic salt. The buffered neutral or alkaline aqueous medium which makes up part of the eluant preferably is the same medium used in applying the insulin to the anion exchanger gel. The inorganic salt, which represents the other component of the eluant, can be any of a wide variety of salts. Typical inorganic salts are sodium chloride, potassium chloride, ammonium chloride, and the like. However, the preferred salt is sodium chloride.

The eluting medium, comprising a mixture of the buffered neutral or alkaline aqueous medium and the inorganic salt, is brought into contact with the anion exchanger gel, and the insulin is eluted therefrom. The inorganic salt is provided as a salt gradient such that the concentration of salt in the buffered neutral or alkaline aqueous medium delivered to the anion exchange gel increases on a step-wise or a continuous basis. The salt concentration generally is increased progressively over a range which is within the range of from zero to about 0.2 mole per liter, or, preferably, to about 0.13 mole per liter, or, most preferably, until elution of the insulin has been achieved. The latter generally will occur before either of the aforementioned upper limits of salt concentration has been reached.

The insulin, eluted from the anion exchanger gel and having a diminished proinsulin and proinsulin-like protein content, then is further purified as aforedescribed using a cation exchanger gel. The insulin can be recovered from the eluate prior to application to the cation exchanger gel, or the eluate, lowered to the appropriate pH by addition of dilute acid, such as dilute hydrochloric acid, can itself be used in applying the insulin to the cation exchanger gel.

In both the cation and anion exchanger gel treatments, the course of elution is followed by any convenient means. A particularly useful method consists of measuring the ultraviolet absorption at 280 mµ of each fraction of eluate collected. Those fractions containing insulin of desired purity are combined and the insulin is recovered therefrom. The process of this invention additionally can be combined as desired with other purification procedures. For example, the purity of the insulin recovered from the cation exchanger gel process of this invention can be further enhanced by subjecting it to a second cation exchanger gel treatment. Additionally, or alternatively, the insulin recovered from the process of this invention can be subjected to a gel filtration treatment such as those described in United States Patents Nos. 3,876,623 and 3,878,186.

Zinc insulin currently represents the most important form of commercial insulin. Consequently, the high purity insulin obtained by the process of the present invention usually will be converted to zinc insulin. Conversion to zinc insulin preferably is made at pH 6.0 in the presence of ammonium acetate buffer. It is not necessary to isolate the insulin from the eluting medium, or to concentrate the solution of purified insulin before crystallizing with zinc. An advantage of the present invention is that the insulin in solution as eluted is of sufficient concentration such that salt or isoelectric precipitations are not required, although such procedures can be employed, if desired.

The zinc insulin thus obtained is exceptionally pure. Its purity can be demonstrated by several methods. The zinc insulin can be analyzed directly for noninsulin protein components such as proinsulin and glucagon. The presence of high molecular weight proteins, which typically include protaminase, a proteolytic enzyme, can be determined by measuring the stability of protamine insulin suspension at elevated temperatures; if protaminase is present, the protamine insulin complex will dissociate because of protamine degradation. Physical properties. such as solubility of the zinc insulin at neutral pH and color of the resulting solution, can be measured. Finally, the specific activity of the insulin can be measured by biological and immunological assays.

The process of this invention can be illustrated by the following examples. These examples are intended for illustrative purposes only and are not to be interpreted as limiting upon the scope of the invention.

## Example 1

To 50 ml. of cold 0.5N acetic acid containing a 7M concentration of urea and having a pH of 3.6 were added 500 mg. of beef zinc insulin crystals which had been partially purified by DEAE-Sephadex anion-exchange chromatography. Analysis of the solution indicated an insulin concentration of 266 units/ml. (13,284 units), a proinsulin concentration of 131 mcg/ml. (6,535 mcg.; 13,070 ppm.), and a total protein concentration as measured by optical density (280 m$\mu$) of 9.73 units/ml.

The solution was applied to a 1.5 x 45 cm. column of SP-Sephadex C-25 previously equilibrated at 5°C. in 7M urea-0.5N acetic acid, pH 3.6. The column was eluted with a mixture of the urea-acetic acid buffer and a sodium chloride gradient of 0.0-0.25M. Fractions of 10-15 ml. each were collected and analyzed by measurement of the percent transmittance at 280 m$\mu$.

The fractions were pooled to obtain five major fractions and analyzed for insulin, pro-insulin, and optical density. The purified insulin fraction (fraction 4) contained 78.26 percent of the insulin, 74.5 percent of the optical density units, and had a proinsulin content of 484 ppm.

### Example 2

To 200 ml. of 0.5N acetic acid containing a 7M concentration of urea and having a pH of 3.6 were added 25.0 gms. of beef sodium insulin. Analysis of the solution indicated 632,687 units of insulin, 509,639 mcg. (20,385 ppm.) of proinsulin, and a total protein content of 21,970 optical density units (280 mµ).

The solution was applied to a 2.5 x 90 cm. column of SP-Sephadex C-25 which had been previously equilibrated in 7M urea-0.5N acetic acid-0.05M sodium chloride, pH 3.6 at 5°C. The column was eluted at 1.0 ml./min. using a mixture of the urea-acetic acid buffer and sodium chloride gradients of 0.05-0.225M and 0.225-0.36M. Fractions were collected and analyzed for proinsulin and optical density at 280 mµ. The fractions were pooled into seven major fractions and assayed for insulin, proinsulin, and optical density. The major insulin fraction contained 94.78 percent of the insulin units, 90.4 percent of the optical density units, and only 9.6 percent of the proinsulin (2917 ppm).

## Example 3

A portion of the beef insulin fraction obtained from Example 2 was diluted with an equal volume of water and adsorbed onto 180 ml. of fresh SP-Sephadex C-25 (10 ml. of swollen gel per gm. of O.D. 280.)

The material contained about 404,220 units of insulin and 47,636 mcg. of proinsulin (2917 ppm). The gel was washed with water and then re-slurried in 7M urea-0.5N acetic acid, pH 3.6 and layered over a column of SP-Sephadex C-25 (2.5 x 90 cm) which had been equilibrated at 5°C. in the same urea-acetic acid buffer. The column was eluted with a mixture of 7M urea-0.5N acetic acid, pH 3.6, and sodium chloride gradients of 0.05-0.225M and 0.225-0.36M. Fractions were collected and analyzed for percent transmittance, optical density, and proinsulin. On the basis of these analyses, certain fractions were pooled. Analysis of that pool containing the major insulin component indicated 351,717 units of insulin and 19 mcg. of proinsulin (1 ppm).

## Example 4

DEAE-Sephadex A-25 (4.5 kg.) was swollen, freed from fine particles, and equilibrated in a buffer containing 0.01M tris(hydroxymethyl)aminomethane, 0.001M ethylenediamine tetraacetate, 7.0M urea, and sufficient hydrochloric acid to obtain pH 8.1. The DEAE-Sephadex A-25 so prepared was packed into a 21.5 x 85 cm. chromatographic column.

Partially purified salt-free porcine insulin (1.9 kg.), containing approximately 10,000 ppm. proinsulin, was dissolved in the pH 8.1 buffer, and the solution was clarified by filtration. The filtrate then was applied to the DEAE-Sephadex A-25 column.

The column was eluted using (1) the pH 8.1 buffer containing 0.02M sodium chloride, (2) a mixture of the pH 8.1 buffer and a sodium chloride gradient from 0.02-0.06M sodium chloride, and (3) a mixture of the pH 8.1 buffer and a sodium chloride gradient of 0.06-0.13M sodium chloride. Elution of the insulin, its precursors, and other pancreatic hormones was monitored in the column eluant fractions by specific radioimmunoassays, polyacrylamide electrophoresis, high pressure liquid chromatography, and ultraviolet light absorption.

Purified insulin, eluted in the last gradient, was acidified to pH 3.6 with hydrochloric acid. Analysis of the recovered insulin in a pool which represented greater than 88% protein recovery indicated a reduction in proinsulin content to less than 300 ppm.

A portion of the acidified solution (14.01.), containing 720 gms. of insulin, was further purified by chromatography on SP-Sephadex C-25. SP-Sephadex C-25 (1.5 kg.) was swollen, freed from fine particles, and equilibrated in a buffer containing 0.1M acetic acid, 7.0M urea, and sufficient hydrochloric acid to provide pH 3.6. The prepared SP-Sephadex C-25 was packed into a 10 x 90 cm. chromatographic column. The insulin solution was applied to the column, and the column was eluted with a mixture of the foregoing buffer and a sodium chloride gradient of 0.15-0.35M.

Highly purified porcine insulin was located in eluant fractions by the foregoing noted techniques. Selected fractions were pooled, and the pool was divided into two fractions. One was freed from urea by gel filtration on Sephadex G-25 and the other on Sephadex G-50, employing, respectively, 0.5N and 1.0N acetic acid solutions. Both final urea-free pools were crystallized as zinc insulin. Assay of the insulin showed:

(a) in the case of the Sephadex G-25 filtration product, a 98% recovered fraction exhibited a further reduction in proinsulin content to 184 ppm., a glucagon content of 1.4 ppm., a pancreatic polypeptide content of 5 ppm., and a somatostatin content of 9 ppb.; and

(b) in the case of the Sephadex G-50 filtration product, a 78% recovered fraction exhibited a further reduction in proinsulin content to 0.8 ppm., a glucagon content of 1.4 ppm., a pancreatic polypeptide content of 0.3 ppm., and a somatostatin content of 7 ppb.

### Example 5

Partially purified, salt-free insulin of bovine origin (2.3 kg.) was dissolved in the pH 8.1 buffer described in Example 4. The insulin was applied to a freshly prepared 21.5 x 80 cm. column of DEAE-Sephadex A-25, which had been equilibrated in the pH 8.1 buffer. The starting bovine insulin preparation contained greater than 20,000 ppm. of proinsulin, approximately 500 ppm. of pancreatic polypeptide, approximately 400 ppm. of glucagon, and greater than 400 ppb. of somatostatin. The column was eluted using (1) 8.1 buffer containing 0.02M sodium chloride, (2) a mixture of the pH 8.1 buffer and a sodium chloride gradient of 0.2-0.6M, and (3) a mixture of the pH 8.1 buffer and a sodium chloride gradient of 0.6M-0.13M. Elution of the insulin, its precursors, and other pancreatic hormones was monitored in the column eluant fractions by specific radioimmunoassays, polyacrylamide electrophoresis, high pressure liquid chromatography, and ultraviolet light absorption. Purified insulin, eluted in the last gradient, was acidified to pH 3.6 with hydrochloric acid. Analysis of the insulin indicated, in a specific pool, 81% of recovered protein containing a proinsulin content of 11,000 ppm.

A portion of the acidified solution (15.0 l.), containing 770 gms. of insulin, was applied to a freshly prepared 10 x 90 cm. column of SP-Sephadex C-25, equilibrated in the pH 3.6 buffer described in Example 4. The column was eluted with a mixture of the pH 3.6 buffer and a sodium chloride gradient of 0.15-0.35M. Highly purified bovine insulin was collected, freed from urea by gel filtration on Sephadex G-50 in 1.0N acetic acid, and crystallized as zinc insulin. Analysis of the insulin indicated a recovery of 75% of the protein in a major pool which had a proinsulin content of less than 40 ppm.

## Example 6

A beef insulin preparation of 5.75 gm. of protein containing 156,839 units of insulin and 295,750 mcg. of proinsulin (51,390 ppm) was dissolved in 91 ml. of 7M Urea-0.1M acetic acid-0.1M NaCl. The mixture was adjusted to pH 3.6 with 10% HCl. The solution was applied to a 1.8 x 45 cm. column of SP-Sephadex C-25 (115 ml. bed volume) previously equilibrated in the same buffer at 5°C. The column was eluted with a sodium chloride gradient comprised of 250 ml. of 0.15M salt in buffer fed by 2000 ml. 0.35M salt in buffer at a flow rate of 1.01 ml/min. The protein elution profile of the effluent fractions was measured by % transmittance. The effluent fractions were pooled into three fractions (A, B, and C) and assayed for insulin, proinsulin, and protein. The major insulin-containing fraction (C) showed a 82.4% reduction in the proinsulin content (9,061 ppm) and the presence of 100.6% of the insulin units.

## Example 7

A beef insulin preparation of 5.75 gm. of protein containing 158,340 units of insulin and 260,400 mcg. of proinsulin (45,208 ppm) was dissolved in 84 ml. of 7M Urea-0.1M acetic acid-0.1M NaCl. The mixture was adjusted to pH 4.2 with 10% HCl. The solution was applied to a 1.8 x 45 cm. column of SP-Sephadex C-25 (115 ml. bed volume) previously equilibrated in the same buffer at 5°C. The column was eluted with a sodium chloride gradient comprised of 250 ml. of 0.15M salt in buffer fed by 2000 ml. 0.35M salt in buffer at a flow rate of 1.06 ml/min. The protein elution profile of the effluent fractions was measured by % transmittance. The effluent fractions were pooled into three fractions (A, B, and C) and assayed for insulin, proinsulin, and protein. The major insulin-containing fraction (C) showed a 60.4% reduction in the proinsulin content (17,937 ppm) and the presence of 85.6% of the insulin units.

### Example 8

A beef insulin preparation of 5.75 gm. of protein containing 153,000 units of insulin and 292,740 mcg. of proinsulin (50,788 ppm) was dissolved in 84 ml. of 7M Urea-0.1M acetic acid-0.1M NaCl. The mixture was adjusted with 10% HCl to pH 3.6. The solution was applied to a 1.8 x 45 cm. column of CM-Sephadex C-25 (115 ml. bed volume) previously equilibrated in the same buffer at 5°C. The column was eluted with a sodium chloride gradient comprised of 250 ml. of 0.15M salt in buffer fed by 2000 ml. 0.35M salt in buffer at a flow rate of 0.73 ml/min. The protein elution profile of the effluent fractions was measured by ·% transmittance. The effluent fractions were pooled into three fractions (A, B, and C) and assayed for insulin, proinsulin, and protein. The major insulin-containing fraction (C) showed a 37.1% reduction in the proinsulin content (31,963 ppm) and the presence of 94% of the insulin units.

## Example 9

A beef insulin preparation of 5.75 g. of protein containing 168,652 units of insulin and 264,000 mcg. of proinsulin (45,754 ppm) was dissolved in 84 ml. of 7M Urea-0.1M acetic acid-0.1M NaCl. The mixture was adjusted with 10% HCl to pH 4.2. The solution was applied to a 1.8 x 45 cm. column of CM-Sephadex C-25 (115 ml. bed volume) previously equilibrated in the same buffer at 5°C. The column was eluted with a sodium chloride gradient comprised of 250 ml. of 0.15M salt in buffer fed by 2000 ml. 0.35M salt in buffer at a flow rate of 0.73 ml/min. The protein elution profile of the effluent fractions was measured by % transmittance. The effluent fractions were pooled into three fractions (A, B, and C) and assayed for insulin, proinsulin and protein. The major insulin-containing fraction (C) showed a 35.0% reduction in the proinsulin content (29,615 ppm) and the presence of 90% of the insulin units.

## Example 10

A pork insulin preparation containing 216.0 x $10^6$ units of insulin, 8,968 g. of protein, and 129 x $10^6$ mcg. of proinsulin (14,384 ppm) was dissolved in 76 l. of cold tris buffer with EDTA and 7M urea. The mixture was adjusted to pH 8.1 with 1000 ml. 10% NaOH. The solution was filtered with 7 kg. of hyflo supercel through a plate and frame filter precoated with 1 kg. of hyflo supercel in the same buffer. The press was washed with 10 l. of cold buffer, and the wash was

X-5317A                          -25-

added to the sample.  The sample charge solution was applied to an Amicon column (365 x 100 cm.) containing 100 l. of DEAE Sephadex A-25 which had been equilibrated at 5°C. using the same buffer.  After application of the charge, 40 l. of buffer containing 0.04M NaCl was applied.  The column then was eluted with a sodium chloride gradient developed by feeding 80 l. of 0.04M salt in buffer with 200 l. of 0.13M salt in buffer. Fractions designated A, B, D1, MS, and D2 were collected.  Samples were assayed for insulin, proinsulin, and protein ($OD_{276}$).  The recoveries were as follows:

| Fraction | Liters | Insulin, % | $OD_{276}$, % | Proinsulin, % (ppm) |
|---|---|---|---|---|
| A | 120 | 0.03 | 1.2 | 0.8 (11,000) |
| B | 135 | 1.2 | 7.0 | 47.2 (91,000) |
| D1 | 18 | 7.4 | 8.1 | 19.4 (33,000) |
| MS | 144 | 79.5 | 68.3 | 29.4 (5,700) |
| D2 | 61 | 5.7 | 5.0 | 0.8 (1,700) |

The mainstream (MS) fraction, 144 l., was adjusted to pH 3.6 with 3400 ml. of 10% HCl and applied to an Amicon column (365 x 100 cm.) containing 100 l. of CM-Sephadex C-25 which had been equilibrated in 0.1N acetic acid-7M urea buffer containing 0.1M NaCl, pH 3.6 at 5°C.  The column was eluted with a sodium chloride gradient developed by feeding 80 l. 0.1N acetic acid-7M urea-0.15M NaCl with 300 l. of 0.1M acetic acid-7M urea-0.3M NaCl at pH 3.6.  Two fractions designated C-1 and MS were collected, and samples were assayed for insulin, proinsulin, and protein ($OD_{276}$). The recoveries were as follows.

| Fraction | Liters | Insulin, % | $OD_{276}$, % | Proinsulin, ppm |
|---|---|---|---|---|
| C-1 | 10 | 8.5 | 7.0 | 5,000 |
| MS | 72 | 107.0 | 81.5 | 1,200 |

## CLAIMS

1. A process for increasing the purity of insulin, which comprises

a) swelling and equilibrating a cross-linked dextran gel cation exchanger containing sulfoalkyl or carboxymethyl groups;

b) packing a column with the swollen gel;

c) dissolving a partially purified insulin at a concentration in the range of from about 0.5% to about 12%, weight per volume, in an acidic aqueous medium having a pH of from about 2.5 to about 4.5 and containing urea in a concentration of from about 4 to about 8 moles per liter of medium;

d) bringing the insulin solution into contact with the column-packed cation exchanger gel; and

e) eluting insulin from the cation exchanger gel at a temperature of from about 5°C. to about 25°C. by passing an eluant over the insulin-containing cation exchanger gel, said eluant comprising acidic aqueous medium containing inorganic salt, and said inorganic salt being present in said acidic aqueous medium in a progressively increasing concentration commencing at or below about 0.2 moles per liter and extending to as high as about 0.4 moles per liter.

2. Process of claim 1, in which the cross-linked dextran gel contains carboxymethyl or sulfopropyl groups.

3. Process of claim 2, in which the concentration of the insulin in the acidic aqueous medium is about 5%, weight per volume.

4. Process of claim 2, in which the pH of the acidic aqueous medium is from about 3.2 to about 4.2.

5. Process of claim 4, in which the pH of the acidic aqueous medium is about 3.6.

6. Process of claim 2, in which the acid in the acidic aqueous medium is acetic acid, hydrochloric acid, or phosphoric acid.

7. Process of claim 6, in which the acid is acetic acid.

8. Process of claim 2, in which urea is present in the acidic aqueous medium at a concentration of from about 7 to about 8 moles per liter.

9. Process of claim 8, in which the urea is present at a concentration of about 7 moles per liter.

10. Process of claim 2, in which the elution is carried out at a temperature of about 5°C.

11. Process of claim 2, in which the inorganic salt is sodium chloride.

12. Process of claim 11, in which the concentration of inorganic salt in the eluant extends to as high as about 0.36 moles per liter.

13. Process of claim 2, in which the acidic aqueous medium has a pH of from about 3.2 to about 4.2 and comprises aqueous acetic acid containing urea at a concentration of about 7 moles per liter, the elution is carried out at a temperature of about 5°C., and the eluant comprises a mixture of the acidic aqueous medium and sodium chloride in a progressively increasing concentration commencing at or below about 0.2 moles per liter and extending to as high as about 0.36 moles per liter.

14.  Process of claim 13, in which the pH of the acidic aqueous medium is about 3.6.

15.  A process for increasing the purity of insulin, which comprises

a)  swelling and equilibrating a cross-linked dextran gel anion exchanger;

b)  packing a column with the swollen anion exchanger gel;

c)  dissolving a partially purified insulin at a concentration in the range of from about 0.5% to about 12%, weight per volume, in a neutral or moderately alkaline aqueous medium having a pH of from about 7 to about 9 and containing urea in a concentration of from about 4 to about 8 moles per liter;

d)  bringing the insulin solution into contact with the column-packed anion exchanger gel;

e)  eluting insulin from the anion exchanger gel at a temperature in the range of from about 5°C. to about 10°C. by passing an eluant over the insulin-containing anion exchanger gel, said eluant comprising neutral or moderately alkaline aqueous medium containing inorganic salt, and said inorganic salt being present in said neutral or moderately alkaline aqueous medium in a progressively increasing concentration extending to as high as about 0.2 moles per liter;

f) swelling and equilibrating a cross-linked dextran gel cation exchanger containing sulfoalkyl or carboxymethyl groups;

g) packing a column with the swollen cation exchanger gel;

h) bringing insulin, obtained from step (e) and dissolved, at a concentration in the range of from about 0.5% to about 12%, weight per volume, in an acidic aqueous medium having a pH of from about 2.5 to about 4.5 and containing urea in a concentration of from about 4 to about 8 moles per liter of medium, into contact with the column-packed cation exchanger gel; and

i) eluting insulin from the cation exchanger gel at a temperature of from about 5°C. to about 25°C. by passing an eluant over the insulin-containing cation exchanger gel, said eluant comprising acidic aqueous medium containing inorganic salt, and said inorganic salt being present in said acidic aqueous medium in a progressively increasing concentration commencing at or below about 0.2 moles per liter and extending to as high as about 0.4 moles per liter.

16. Process of claim 15, in which the pH of the acidic aqueous medium is from about 3.2 to about 4.2 and the pH of the neutral or alkaline aqueous medium is about 8.0 to about 8.5.

17.  Process of claim 16, in which the pH of the acidic aqueous medium is about 3.6 and the pH of the neutral or alkaline aqueous medium is about 8.1.

18.  Process of claim 15, in which the acid in the acidic aqueous medium is acetic acid, hydrochloric acid, or phosphoric acid.

19.  Process of claim 15, in which

(a)  in the treatment of insulin using an anion exchanger gel, the neutral or alkaline aqueous medium has a pH of from about 8.0 to about 8.5 and contains urea at a concentration of about 7 moles per liter, the elution is carried out at a temperature of about 5°C., and the eluant comprises neutral or alkaline aqueous medium containing sodium chloride, said sodium chloride being present in said neutral or moderately alkaline aqueous medium in a progressively increasing concentration extending to as high as about 0.13 moles per liter; and

(b)  in the treatment of insulin using a cation exchanger gel, the acidic aqueous medium has a pH of from about 3.2 to about 4.2 and comprises aqueous acetic acid containing urea at a concentration of about 7 moles per liter, the elution is carried out at a temperature of about 5°C., and the eluant comprises acidic aqueous medium containing sodium chloride, said sodium chloride being present in said acidic aqueous medium in a progressively increasing concentration extending to as high as about 0.36 moles per liter.

20. Process of claim 19, in which the pH of the neutral or alkaline aqueous medium is about 8.1.

21. Process of claim 19, in which the pH of the acidic aqueous medium is about 3.6.

22. Insulin purified by a process according to any of the preceding claims.

European Patent
Office

**EUROPEAN SEARCH REPORT**

0013826
Application number

EP 79 30 3005

| Category | Citation of document with indication, where appropriate, of relevant passages | Relevant to claim |
|---|---|---|
| A | DE - A1 - 2 757 377 (THE REGENTS OF THE UNIVERSITY OF MINNESOTA) <br> * claims 1 and 14 * <br> -- | 1,2 |
| A,D | US - A - 3 876 623 (R.L. JACKSON et al.) <br> * claims 1, 6 and 11 * <br> -- | 1,4, 11 |
| A,D | US - A - 3 878 186 (R.L. JACKSON) <br> * claims 1, 4, 14 and 9 * <br> ---- | 1,4, 7,11 |

**DOCUMENTS CONSIDERED TO·BE RELEVANT**

**CLASSIFICATION OF THE APPLICATION (Int. Cl.³)**

C 07 C 103/52
A 61 K   37/26
//B 01 D 15/04
   B 01 J 39/22

**TECHNICAL FIELDS SEARCHED (Int.Cl.³)**

A 61 K   37/26
B 01 D   15/04
B 01 J   39/04
B 01 J   39/22
C 07 C 103/52
C 07 G   7/00
C 07 G   15/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| Berlin | 31-03-1980 | KNAACK |

EPO Form 1503.1  06.78